# EUROPÄISCHE PATENTANMELDUNG

(11) **EP 0 968 689 A2**
(43) Veröffentlichungstag der Anmeldung: **05.01.2000**
(21) Anmeldenummer: 99111554.4
(22) Anmeldetag: 15.06.1999
(51) Int. Cl.: A61F 2/06

(54) **Radial aufweitbare Stützvorrichtung IV**

(30) Priorität: 03.07.1998 DE 19829701
(71) Anmelder: W.C. Heraeus GmbH & Co. KG, 63450 Hanau (DE)
(72) Erfinder: Kock, Wulf Dr., 63755 Alzenau (DE); Herklotz, Günter Dr., 63486 Bruchköbel (DE); Frericks, Matthias, 63456 Hanau (DE); Trötzschel, Jens, 63486 Bruchköbel (DE)
(74) Vertreter: Kühn, Hans-Christian

(57) **Zusammenfassung**

Es wird eine radial aufweitbare Stützstruktur bereitgestellt zur Offenhaltung von Lumina innerhalb eines Körpers, insbesondere eines Blutgefäßes, mit einem rohrförmigen, mindestens zwei Teilstrukturen aufweisenden Körper mit einer sich zwischen einem ersten und einem zweiten Ende erstreckenden Wandfläche, die mehrere Ausschnitte, insbesondere Schlitze aufweist, die im wesentlichen parallel zu der Längsachse des rohrförmigen Körpers ausgerichtet sind,
- wobei mindestens eine Teilstruktur (11) ohne Unterbrechung in axialer Richtung zumindest nahezu vom ersten bis zum zweiten Ende des rohrförmigen Körpers verläuft, die erste Teilstruktur (1, 2) zumindest in radialer Richtung dehnbar ist und mindestens einen Radial-Dehnungselement-Typ aufweist, wobei die einzelnen Radial-Dehnungselemente (12, 13) als Helix oder helixartig angeordnet sind und die zweite Teilstruktur (11) in axialer Richtung nahezu starr ist und/oder
- wobei beide Teilstrukturen (1, 2; 11) derart angeordnet sind, daß bei radialer Aufweitung des rohrförmigen Körpers die zweite Teilstruktur (11) die dabei auftretenden längsaxialen Kräfte aufnimmt.

## Beschreibung

Die Erfindung betrifft eine radial aufweitbare Stützstruktur zur Offenhaltung von Lumina innerhalb eines Körpers, insbesondere eines Blutgefäßes.

EP 0 335 341 B1 betrifft u. a. ein aufweitbares intraluminares vaskuläres Gewebe oder Prothese mit zumindest einem dünnwandigen rohrförmigen Teil mit ersten und zweiten Enden und einer zwischen dem ersten und zweiten Ende angeordneten Wandfläche, die eine im wesentlichen gleichförmige Dicke und mehrere Schlitze aufweist, die im wesentlichen parallel zur Längsachse des rohrförmigen Teils ausgerichtet sind, wobei das rohrförmige Teil einen ersten Durchmesser aufweisen kann, der den intraluminalen Transport des rohrförmigen Teils in einen ein Lumen aufweisenden Körperdurchgang ermöglicht, und wobei das rohrförmige Teil einen zweiten, aufgeweiteten und deformierten Durchmesser aufweisen kann, nach dem vom Inneren des rohrförmigen Teils aus eine radial nach außen gerichtete Kraft aufgebracht ist, wobei der zweite Durchmesser variabel ist und vom Betrag der auf das rohrförmige Teil ausgeübten Kraft abhängt, wodurch das rohrförmige Teil aufgeweitet und deformiert wird, um das Lumen des Körperdurchgangs aufzuweiten. Das vaskuläre Gewebe oder die vaskuläre Prothese weist mehrere rohrförmige Teile und zumindest ein Verbindungsteil auf, welches zwischen aneinander angrenzenden rohrförmigen Teilen angeordnet ist, um aneinander angrenzende rohrförmige Teile biegbar miteinander zu verbinden.

Nachteilig bei diesem intraluminaren vaskulären Gewebe ist die relativ hohe radiale Steifigkeit der einzelnen Teile, was die in einem Lumen notwendige Flexibilität zur Verhinderung von inneren Verletzungen merklich beeinträchtigt.

In US 4,969,458 wird eine intraluminare Stützstruktur offenbart, die aus einer spiralförmigen Feder besteht. Nachteilig hierbei ist die relativ hohe radiale Instabilität, die zu unerwünschten Knickstellen führen kann.

Aus dem Vorgenannten ergibt sich das Problem, mit Hilfe einer neuartigen Stützstruktur die oben genannten Nachteile zumindest teilweise zu beseitigen. Das sich ergebende Problem liegt insbesondere darin, eine hohe Stabilität in Längsrichtung bei nicht zu geringer radialer Steifigkeit unter Vermeidung einer Verkürzung der Stützstruktur bei radialer Aufweitung und unter Vermeidung von unkontrollierten radialen Knickbewegungen zu gewährleisten.

Dieses Problem wird erfindungsgemäß durch eine Stützstruktur nach Anspruch 1 gelöst.

Die erfindungsgemäße Stützstruktur weist einen rohrförmigen, mindestens zwei Teilstrukturen aufweisenden Körper mit einer sich zwischen einem ersten und einem zweiten Ende erstreckenden Wandfläche auf, die mehrere Ausschnitte, insbesondere Schlitze, aufweist, die im wesentlichen parallel zu der Längsachse des rohrförmigen Körpers ausgerichtet sind. Mindestens eine Teilstruktur verläuft ohne Unterbrechung in achsialer Richtung zumindest nahezu vom ersten bis zum zweiten Ende des rohrförmigen Körpers. Die erste Teilstruktur ist zumindest in radialer Richtung dehnbar und weist mindestens einen Radial-Dehnungselement-Typ auf, wobei die einzelnen Radial-Dehnungselemente als Helix oder helixartig angeordnet sind. Die zweite Teilstruktur ist in achsialer Richtung nahezu starr. Der Radial-Dehnungselement-Typ kann geschlossen, beispielsweise oval-, rechteck-, rauten- oder ellipsenförmig, oder offen, beispielsweise mäanderförmig, ausgebildet sein.

Durch insbesondere diese Anordnung der Teilstrukturen wird erreicht, daß bei radialer Aufweitung des rohrförmigen Körpers die zweite Teilstruktur die dabei auftretenden längsaxialen Kräfte aufnimmt. Dies hat zur Folge, daß die radiale von der axialen Deformation unabhängig ist und somit keine Verkürzung in Längsrichtung bei radialer Aufweitung stattfindet. Dies ist bei der invasiven Chirurgie besonders wichtig, um eine gewünschte Stützwirkung über eine bestimmte Länge bereitzustellen.

In vorteilhafter Weise weist mindestens eine Teilstruktur mindestens ein Mäandermuster auf, um die Flexibilität zu erhöhen. Dies gilt für die mindestens zwei Teilatrukturen.

Es ist vorteilhaft, daß die Schleifen des Mäandermusters unterschiedlich groß sind. Dies bezieht sich auf die Elongation (Amplitude) und/oder "Wellenlänge", sowohl in radialer als auch in Längsrichtung der Stützstruktur. Damit ist insgesamt eine zumindest bezogen auf die erste Teilstruktur hohe homogene Aufweitbarkeit gegeben.

Weiterhin ist es von Vorteil, daß das Mäandermuster ein Doppelmäandermuster ist, um die radiale Aufweitbarkeit zu erhöhen.

Das Doppelmäandermuster besteht vorteilhafterweise aus einem ersten Mäandermuster mit Schleifen und einem zweiten Mäandermuster mit im Vergleich zu den Schleifen des ersten Mäandermusters größeren oder kleineren Schleifen, um auf diese Art und Weise bei radialer aufweitender Beaufschlagung zumindest ein annähernd gleichmäßiges Aufweiten zu erreichen.

Darüber hinaus ist in vorteilhafter Weise ein jeweils an den Längsenden der ersten Radial-Dehnungselemente angreifender Verbindungs-Typ als schleifenförmiger Steg ausgebildet; der Steg verhindert bzw. vermindert bei Biegebeanspruchung ein Abstehen oder Herausspießen der Schleifen aus der Oberfläche der Stützstruktur. Darüber hinaus verbessern sie im aufgeweiteten Zustand die Abstützwirkung des Stents durch eine gleichmäßigere Oberfläche.

Schließlich ist es vorteilhaft, wenn die Wandfläche eine im wesentlichen gleichförmige Dicke aufweist, um die homogene radiale Ausdehnung zu verbessern.

Die Stützstruktur kann beispielsweise aus einem für medizinische Zwecke geeigneten Edelstahl bestehen und/oder eine biokompatible Beschichtung aufweisen. Darüberhinaus kann jedes biokompatible Material als Werkstoff zur Herstellung von Stents in Frage kommen, zum Beispiel Tantal, Platin, Niob, Legierungen und Kunststoffe. Die Strukturen können durch Laserschneiden, Elektroerosion, Ätzen oder auch Spanabheben hergestellt werden.

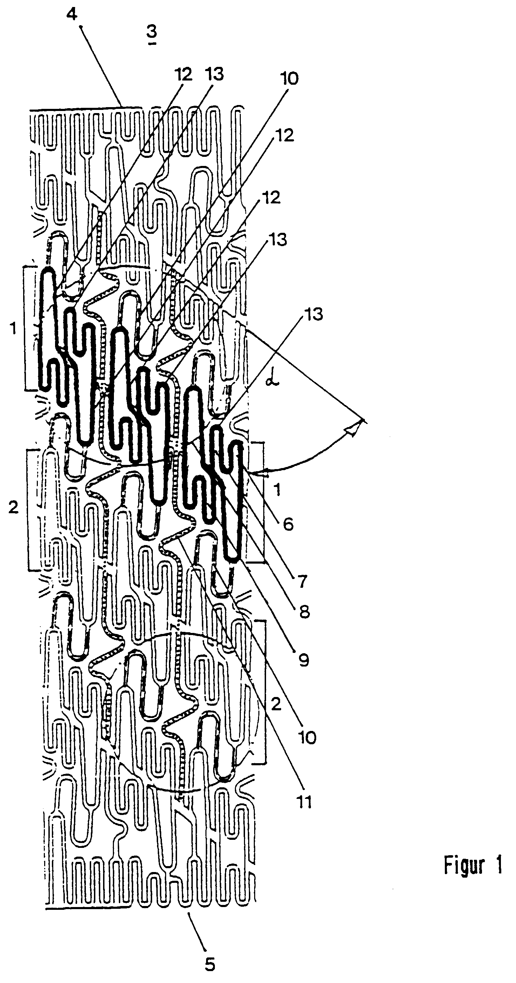

Nachfolgend wird ein Ausführungsbeispiel der Erfindung anhand einer Zeichnung erläutert. In der Zeichnung zeigt
Figur 1 - eine abgerollte Stützstruktur.

Figur 1 zeigt die abgerollte Wandfläche einer Stützstruktur. Diese weist eine erste aus aus Mäanderstrukturen 12 und 13 bestehenden Doppelmäanderstrukturen bestehende Teilatruktur 1, 2 und eine zweite Teilstruktur 11 auf. Die Mäanderstrukturen bilden mehrere Schlitze 6, 7, 8, 9, die im wesentlichen parallel zu der Längsachse des rohrförmigen Körpers 3 ausgerichtet sind. Die zweite Teilstruktur 11 läuft ohne Unterbrechung in axialer Richtung nahezu vom ersten 4 bis zum zweiten Ende 5. Die erste Teilstruktur ist in radialer Richtung dehnbar und weist einen Radial-Dehnungselement-Typ auf, der aus den Radial-Dehnungselementen 12, 13 besteht. Diese sind als Helix angeordnet. Die zweite Teilstruktur 11 ist trotz des schleifenförmigen Verlaufs in axialer Richtung relativ starr.

Weiterhin ist der jeweils an den Längsenden der Radial-Dehnungselemente 12, also in diesem Fall an den Schleifenenden des ersten Mäandermusters 12, angreifender Verbindungstyp 10 als schleifenförmiger Steg ausgebildet.

Der beispielhaft dargestellte Stent weist eine Länge von ca. 15 mm und einen radialen Umfang von ca. 4 mm auf. Der Steigungswinkel α der Helix beträgt, bezogen auf die Längsrichtung der Stützstruktur, ca. 50°.

Mit dieser beispielhaften Ausgestaltung der erfindungsgemäßen Stützstruktur werden ausgezeichnete Ergebnisse erreicht.

## Patentansprüche

1. Radial aufweitbare Stützstruktur zur Offenhaltung von Lumina innerhalb eines Körpers, insbesondere eines Blutgefäßes, mit einem rohrförmigen, mindestens zwei Teilstrukturen aufweisenden Körper mit einer sich zwischen einem ersten und einem zweiten Ende erstreckenden Wandfläche, die mehrere Ausschnitte, insbesondere Schlitze, aufweist, die im wesentlichen parallel zu der Längsachse des rohrförmigen Körpers ausgerichtet sind,
- wobei mindestens eine Teilstruktur (11) ohne Unterbrechung in axialer Richtung zumindest nahezu vom ersten bis zum zweiten Ende des rohrförmigen Körpers verläuft, die erste Teilstruktur (1, 2) zumindest in radialer Richtung dehnbar ist und mindestens einen Radial-Dehnungselement-Typ aufweist, wobei die einzelnen Radial-Dehnungselemente (12, 13) als Helix oder helixartig angeordnet sind und die zweite Teilstruktur (11) in axialer Richtung nahezu starr ist und/oder
- wobei beide Teilstrukturen (1, 2; 11) derart angeordnet sind, daß bei radialer Aufweitung des rohrförmigen Körpers die zweite Teilstruktur (11) die dabei auftretenden längsachsialen Kräfte aufnimmt.

2. Stützstruktur nach Anspruch 1, dadurch gekennzeichnet, daß mindestens eine Teilstruktur (1, 2; 11) mindestens ein Mäandermuster (11; 12, 13) aufweist.

3. Stützstruktur nach Anspruch 2, dadurch gekennzeichnet, daß die Schleifen des Mäandermusters unterschiedlich groß sind.

4. Stützstruktur nach Anspruch 2 oder 3, dadurch gekennzeichnet, daß das Mäandermuster ein Doppelmäandermuster ist.

5. Stützstruktur nach Anspruch 4, dadurch gekennzeichnet, daß das Doppelmäandermuster aus einem ersten Mäandermuster (12) mit Schleifen und einem zweiten Mäandermuster (13) mit im Vergleich zu den Schleifen des ersten Mäandermusters größeren oder kleineren Schleifen besteht.

6. Stützstruktur nach einem der vorhergehenden Ansprüche, dadurch gekennzeichnet, daß mindestens ein jeweils an den Längsenden der ersten Radial-Dehnungselemente (12) angreifender Verbindungs-Typ (10) als schleifenförmiger Steg ausgebildet ist.

7. Stützstruktur nach einem der vorhergehenden Ansprüche, dadurch gekennzeichnet, daß die Wandfläche eine im wesentlichen gleichförmige Dicke aufweist.
